# EUROPEAN PATENT SPECIFICATION

(11) **EP 1 302 250 B1**
(45) Date of publication and mention of the grant of the patent: **27.04.2005**
(21) Application number: 01830649.8
(22) Date of filing: 16.10.2001
(51) Int. Cl.: B08B 9/027, B08B 3/02, A61L 2/20

(54) **An apparatus and process for washing and sterilising industrial plants**
Vorrichtung und Verfahren zum Waschen und Sterilisieren von industriellen Anlagen
Dispositif et procédé de lavage et de stérilisation d'installations industrielles

(43) Date of publication of application: 16.04.2003
(73) Proprietor: Ico Oleodinamici S.p.A., 41010 Frazione San Damaso (MO) (IT)
(72) Inventor: Barani, Ruggero, 41100 Modena (IT)
(74) Representative: Gotra, Stefano

(56) References cited:
- EP-A- 0 522 160
- DE-A- 3 642 611
- NL-A- 288 009
- US-A- 5 329 950
- US-A- 5 348 058

## Description

Especially, though not exclusively, the invention is usefully applied in plants in the pharmaceutical industry, such as for example granulation, mixing, dissolving, coating solid particles, bins, tanks etc.

US 5 329 950 discloses a self-containing cleaning and sanitizing equipment which includes: a first liquid holding tank for a cleaning solution, a second liquid holding tank for a sanitizing solution, a cleaning line running from the first tank and a sanitizing line running from the second tank. A sterilizing gas is introduced in the sanitizing line.

The main aim of the present invention is to provide an especially efficient process for automatic washing, drying and sterilising of the treated surfaces of an industrial plant.

An advantage is that even the parts which do not come into direct contact with the washing liquid spraying the plant during cleaning operations are fully sterilised.

A further aim is to provide a simple and economical apparatus for realising the above-cited process.

A further aim of the invention is simply and practically to sterilise the apparatus using cold fluids.

These aims and advantages and others besides are all attained by the invention as it is characterised in the appended claims.

Further characteristics and advantages of the present invention will better emerge from the detailed description that follows of a preferred but non-exclusive embodiment of the invention, illustrated purely by way of a nonlimiting example in the accompanying figure of the drawing, in which:

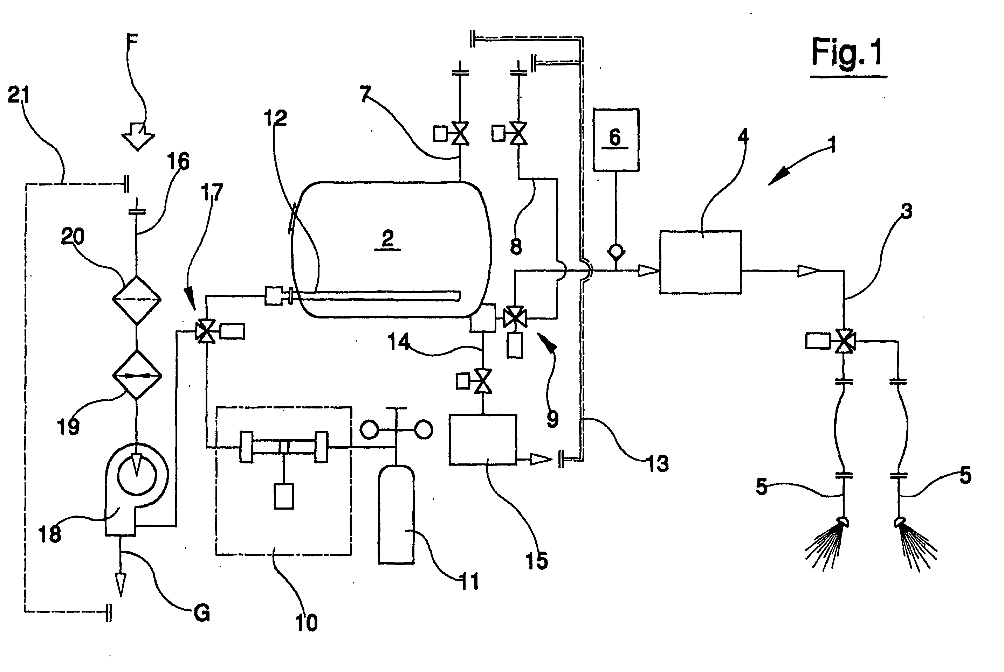

figure 1 is a diagram of an apparatus realised according to the invention.

With reference to the figure of the drawing, 1 denotes in its entirety an apparatus for washing and sterilising industrial plants comprising a tank 2 containing a washing liquid (for example, water) connected, through a high-pressure feed line 3 comprising a pipe including a pump 4 (for example a 70-bar pressure pump), to one or more washing terminals 5 (for example water nozzles) which are associated with the surfaces of the plant which are to be treated. In the illustrated embodiment there is also a batcher 6 which feeds the line 3 with measured amounts of a detergent at a point upstream of the pump 4.

A line 7 is attached to the tank 2, which will join up with an external line supplying the washing fluid, for example the municipal water supply. A further line 8, also removably connectable to the water supply, joins the high-pressure feed line 3 at a three-way switch valve 9 predisposed (upstream of the high-pressure pump and at a non-pressurised point) selectively to connect the high-pressure feed line 3 with the tank supply 2 or with the municipal water supply through the connecting line 8. Both connecting lines 7 and 8 are provided with an intercept valve.

The apparatus 1 comprises means for introducing a sterilising gas into the tank 2, which gas mixes with the washing liquid. The means for introducing the sterilising gas comprise a gas feed line which includes an ozoniser 10 (provided with a high-voltage generator). The ozoniser 10 takes oxygen from a tank 11 and transforms it into ozone. Any fixed supply line of oxygen can be used. The oxygen supply line terminates inside the tank 2 with an injector 12 located close to the bottom of the tank 2 which releases the ozone (a sterilising gas) into the washing liquid, thus obtaining a mixture of the two substances.

The apparatus comprises a secondary sterilising line 13 predisposed to connect selectively the draining pump 15 of the tank 2 with connecting lines 7 and 8, so that these parts too can be sterilised. In the illustrated embodiment these two water lines 7 and 8 supply the washing liquid (water) to the tank, and the high-pressure feed line 3. During sterilisation the ends of the lines 7 and 8 are detached from the municipal water supply and joined up to the secondary automatic sterilising line 13. The secondary line 13 exhibits at one end a removable and selective connection with the lines 7 and 8; while at its other end it exhibits a connector for selective and removable connection with a subsidiary draining line 14 of the tank 2, provided with the draining pump 15, which can on demand be used both for draining liquid remaining in the tank 2 and for automatic sterilisation of parts of the apparatus.

The apparatus 1 further comprises a drying line 16 predisposed for supplying a drying fluid over the treated surfaces. The drying line 16 is connected through a three-way switch valve 17 to the feed line which introduces the sterilising gas into the tank; this connection enables the sterilising gas - ozone - to be mixed with the drying gas - air. The drying line 16 is provided with a feed line which removes air from the atmosphere; along the feed line means for moving the air are arranged (for example an aspirating fan), a heater 19 and a filter 20. As for the lines 7 and 8, a secondary line 21 is provided for the drying line 16, is selectively connected to the drying line 16 and realises a closed auto-sterilising circuit for the drying line. The secondary recycling lines 13 and 21 function as by-passes so that when so desired the short lines 7 and 8 can be auto-sterilised, as well as the drying line 16.

The various above-mentioned parts of the apparatus 1 - in particular the tank 2, the high-pressure feed line 3, the pump 4, the washing terminals 5 and the means for introducing sterilising gas, but also the drying system as well as the auto-sterilising systems with ozonised water and ozonised air - are mounted on a mobile washing unit with wheels (not illustrated).

The apparatus enables automatic sterilisation and cleaning of structural components with no need to dismount them: this is realised by means of the on-site production of sterilising gas (ozone) which is mixed with the washing liquid and thus conveyed at low temperature to the inside of the hydraulic circuit of the apparatus. The same solution is applied to the pneumatic drying circuit, with the difference that the sterilising gas is mixed with the drying gas. This solution means that the same control unit can be programmed to command both the washing/drying operations and the automatic sterilising cycle.

Cold automatic sterilising (instead of, for example, sterilising using heated steam) means that relatively-cheap parts of the apparatus itself can be used in the operation, as no compatibility with high temperatures is required.

Production and introduction of sterilising gas into the tank 2 are controlled (using known techniques) so that the saturation level of sterilising gas (in this embodiment ozone) mixed with the washing liquid (in this case water) in the tank 2 is never exceeded, so no over-production of ozone obtains.

It has been seen that by using a high-pressure washing liquid and a mixed-in sterilising gas (i.e. in the present embodiment ozonised water) an extremely efficient wash and sterilisation is obtained of all of the treated surfaces of the plant. In the case of ozonised water, high-pressure delivery over the surfaces to be treated accelerates the transformation of the ozone contained in the water into active oxygen, which is able to sterilise even those parts of the machine which are not directly sprayed by the jets of pressurised liquids.

## Claims

1. An apparatus for washing and sterilising industrial plants, comprising at least one tank (2) for a washing liquid connected, through at least one high-pressure feed line (3) equipped with at least one pump (4), to at least one washing terminal (5) which is associated to a surface to be treated, means (10, 11, 12) for introducing a sterilising gas into the tank (2), which sterilising gas mixes with the washing liquid in the tank, **characterised in that** it comprises at least one secondary line for sterilisation (13) which, via a draining pump (15), can selectively place the tank (2) in communication with lines (7 and 8) for supplying a washing liquid to the tank (2) and respectively to the high-pressure feed line (3), each of the lines (7 and 8) being provided with at least one end which can be attached to an external washing liquid supply source; and **in that** it comprises a drying line (16) for supplying a gaseous drying fluid to treated surfaces, which drying line (16) is connected to means for introducing sterilising gases for mixing the sterilising gas with the gaseous drying fluid;
the apparatus comprising a secondary line (21) for recycling, selectively connected to the drying line in order to realise a sterilising closed circuit of the drying line (16); at least the tank (2), the high-pressure feed line (3), the pump (4), the washing terminal (5) and the means (10, 11, 12) for introducing the sterilising gas being mounted on a compact washing unit which is self-sufficient and mobile on wheels.

2. The apparatus of claim 1, **characterised in that** the means for introducing the sterilising gas comprise an ozoniser (10) which removes oxygen from a tank (11) and transforms the oxygen into ozone, which is conveyed to the tank (2).

3. A process for washing and sterilising industrial plants in which a washing liquid is sourced from a tank (2), is pumped and sent at high pressure towards at least one washing terminal (5) which directs the washing liquid to a plant to be washed and sterilised, and in which a sterilising gas is injected internally of the tank (2), which sterilising gas mixes with the washing liquid, **characterised in that** the process is realised by an apparatus according to any one of claims from 1 to 2, and **in that** the process automatically washes and automatically sterilises, using the washing liquid sourced from the tank (2), at least the connections for connecting the tank (2) and the high-pressure feed line (3) with an external source of the washing liquid; the sterilising gas comprising ozone; after washing, surfaces of the plant being sprayed with a drying flow of a gas containing the sterilising gas; a part of the apparatus being automatically sterilised by the closed-circuit drying flow of gas.

## Patentansprüche

1. Vorrichtung zum Waschen und Sterilisieren von industriellen Anlagen, enthaltend wenigstens einen Behälter (2) für eine Waschflüssigkeit, der über wenigstens eine Hochdruckleitung (3), versehen mit wenigstens einer Pumpe (4), an wenigstens ein Waschterminal (5) angeschlossen ist, letzteres einer zu behandelnden Oberfläche zugeordnet, Mittel (10, 11, 12) zum Einführen eines Sterilisiergases in den Behälter (2), welches Sterilisiergas sich mit der Waschflüssigkeit in dem Behälter vermischt, **dadurch gekennzeichnet, dass** sie wenigstens eine sekundäre Sterilisierleitung (13) enthält, welche über eine Drainagepumpe (15) wahlweise den Behälter (2) mit Leitungen (7 und 8) zum Zuführen einer Waschflüssigkeit in den Behälter (2) und jeweils mit der Hochdruckleitung (3) verbinden kann, wobei jede der Leitungen (7 und 8) mit wenigstens einem Ende versehen ist, welches an eine aussenliegende Quelle für die Zufuhr der Waschflüssigkeit angeschlossen werden kann; und **dadurch**, dass sie eine Trocknungsleitung (16) zur Zufuhr eines gasigen Trocknungsfluids an die behandelten Oberflächen enthält, welche Trocknungsleitung (16) an Mittel zum Einführen von Sterilisiergasen angeschlossen ist, und zwar zum Mischen des Sterilisiergases mit dem gasigen Trocknungsfluid; wobei die Vorrichtung eine sekundäre Leitung (21) zur Wiedergewinnung enthält, die wahlweise an die Trocknungsleitung angeschlossen wird, um einen sterilisierenden geschlossenen Kreis der Trocknungsleitung (16) herzustellen; und wobei wenigstens der Behälter (2), die Hochdruckleitung (3), die Pumpe (4), das Waschterminal (5) und die Mittel (10, 11, 12) zum Einführen des Sterilisiergases an einer kompakten Wascheinheit montiert sind, welche eigenständig und auf Rädern verfahrbar ist.

2. Vorrichtung nach Patentanspruch 1, **dadurch gekennzeichnet, dass** die Mittel zum Einführen des Sterilisiergases einen Ozonisator (10) enthalten, welcher Sauerstoff aus einem Behälter (11) entnimmt und den Sauerstoff in Ozon umwandelt, der in den Behälter (2) geleitet wird.

3. Verfahren zum Waschen und Sterilisieren von industriellen Anlagen, bei welchem eine Waschflüssigkeit aus einem Behälter (2) entnommen, gepumpt und mit hohem Druck an wenigstens ein Waschterminal (5) geleitet wird, welches die Waschflüssigkeit auf eine zu waschende und sterilisierende Fläche richtet, und bei welchem ein Sterilisiergas in das Innere des Behälters (2) eingeführt wird, welches Sterilisiergas sich mit der Waschflüssigkeit vermischt, **dadurch gekennzeichnet, dass** das Verfahren durch eine Vorrichtung nach einem jeden der Patentansprüche von 1 bis 2 realisiert wird, und **dadurch**, dass das Verfahren unter Verwendung der aus dem Behälter (2) entnommenen Waschflüssigkeit wenigstens die Verbindungen für den Anschluss des Behälters (2) und der Hochdruckleitung (3) an eine aussenliegende Quelle der Waschflüssigkeit automatisch wäscht und automatisch sterilisiert; wobei das Sterilisiergas Ozon enthält; wobei nach dem Waschen die Oberflächen der Anlage mit einem Trocknungsfluid eines das Sterilisiergas enthaltenden Gases besprüht wird; und wobei ein Teil der Vorrichtung automatisch durch den geschlossenen Kreis des Trocknungsgasstrom sterilisiert wird.

## Revendications

1. Dispositif de lavage et de stérilisation d'installations industrielles, comprenant au moins un réservoir (2) pour un liquide de lavage relié, par l'intermédiaire d'au moins une ligne d'alimentation à haute pression (3) pourvue d'au moins une pompe (4), à au moins un terminal de lavage (5) associé à une surface à traiter, des moyens (10, 11, 12) pour introduire un gaz stérilisant dans le réservoir (2), lequel gaz stérilisant se mélange avec le liquide de lavage dans le réservoir, **caractérisé en ce qu'**il comprend au moins une ligne secondaire de stérilisation (13) qui, par l'intermédiaire d'une pompe de drainage (15), peut mettre sélectivement le réservoir (2) en communication avec les lignes (7 et 8) pour fournir un liquide de lavage au réservoir (2) et respectivement à la ligne d'alimentation à haute pression (3), chacune des lignes (7 et 8) étant pourvue d'au moins une extrémité qui peut être reliée à une source externe d'alimentation de liquide de lavage; et **en ce qu'**il comprend une ligne de séchage (16) pour fournir un fluide gazeux séchant aux surfaces traitées, laquelle ligne de séchage (16) est reliée à des moyens d'introduction de gaz stérilisants afin de les mélanger au fluide gazeux de séchage;
le dispositif comprend une ligne secondaire (21) de recirculation, sélectivement reliée à la ligne de séchage de manière à réaliser un circuit fermé de stérilisation de la ligne de séchage (16); au moins le réservoir (2), la ligne d'alimentation à haute pression (3), la pompe (4), le terminal de lavage (5) et les moyens (10, 11, 12) d'introduction du gaz stérilisant étant montés sur une unité de lavage compacte autosuffisante et mobile sur des roues.

2. Dispositif selon la revendication 1, **caractérisé en ce que** les moyens d'introduction du gaz stérilisant comprennent un ozoniseur (10) qui élimine l'oxygène d'un réservoir (11) et le transforme en ozone, qui est convoyé vers le réservoir (2).

3. Procédé de lavage et de stérilisation d'installations industrielles, dans lequel un liquide de lavage est fourni par un réservoir (2), est pompé et envoyé à haute pression vers au moins un terminal de lavage (5) qui dirige le liquide de lavage vers une installation à laver et stériliser, et dans lequel un gaz stérilisant est injecté à l'intérieur du réservoir (2), lequel gaz stérilisant se mélange au liquide de lavage, **caractérisé en ce que** le procédé est mis en oeuvre par un dispositif selon n'importe laquelle des revendications 1 à 2, et **en ce que** le procédé lave et stérilise automatiquement, en utilisant le liquide de lavage provenant du réservoir (2), au moins les liaisons pour relier le réservoir (2) et la ligne d'alimentation à haute pression (3) avec une source externe du liquide de lavage; le gaz de stérilisation comprend de l'ozone; après le lavage, les surfaces de l'installation étant vaporisées avec un flux séchant d'un gaz contenant le gaz stérilisant; une partie du dispositif étant automatiquement stérilisée par le flux de gaz séchant en circuit fermé.
